# EUROPEAN PATENT APPLICATION

(11) **EP 3 760 729 A1**
(43) Date of publication of application: **06.01.2021**
(21) Application number: 19761353.2
(22) Date of filing: 27.02.2019
(51) Int. Cl.: C12Q 1/68, A61Q 19/08

(54) **METHOD OF SCREENING FOR ACTIVE INGREDIENT CAPABLE OF PROTECTING TELOMERES IN SKIN CELLS**

(30) Priority: 28.02.2018 CN 201810166961
(71) Applicant: Jala Group Co., Shanghai 200040 (CN)
(72) Inventor: JIANG, Dandan, Shanghai 200040 (CN); DOS SANTOS, Morgan, Shanghai 200040 (CN); JIANG, Shanshan, Shanghai 200040 (CN)
(74) Representative: Lavoix
(86) International application number: PCT/CN2019/076230
(87) International publication number: WO 2019/165962

(57) **Abstract**

The present invention discloses a method for screening an active substance protecting telomeres in skin cells. Protection of the telomeres in skin cells refers to regulating hsa-miR-1290 in skin cells by using the active substance. The present invention reveals, through experiments, that the expression of hsa-miR-1290 is enhanced in the skin subjected to ultraviolet irradiation, and the active substance reducing the expression of hsa-miR-1290 may promote the expression of the CTC1 gene protecting the telomeres, and improve the structure and functionality of the skin.

SEQUENCE LISTING

<110>  JALA Group COMPANY

<120>  METHOD FOR SCREENING ACTIVE SUBSTANCES PROTECTING TELOMERES IN
       SKIN CELLS

<130>  BET 20M3230

<150>  CN201810166961.8
<151>  2018-02-28

<150>  PCT/CN2019/076230
<151>  2019-02-27

<160>  1

<170>  PatentIn version 3.5

<210>  1
<211>  19
<212>  RNA
<213>  Homo sapiens

<400>  1
uggauuuuug gaucaggga                                                    19

## Description

### TECHNICAL FIELD

The present invention pertains to the field of biotechnologies, relates to a method for screening active substances protecting telomeres in skin cells, and more specifically, relates to evaluating a protection effect on the telomeres in skin cells by the active substances by evaluating an expression level of hsa-miR-1290 in skin cells and thus developing an external skin preparation for improving skin aging.

### BACKGROUND

In today's society, people are more and more concerned about their skin conditions, and expect to keep energetic with skin care products. Therefore, various products solving different skin problems are developed. With time elapsed, each consumer may face up with the problem of skin aging. Therefore, anti-aging skin care products have a dominating role in varieties of skin care products.

Skin aging is categorized into endogenous aging and exogenous aging. Endogenous aging refers to aging caused by genetic changes. Exogenous aging refers to aging caused by external factors, for example, ultraviolet irradiation, smoking, environmental pollution and the like. Human skin is inevitably subjected to sunlight irradiation over months or years, and ultraviolet irradiation from the sunlight is the main factor directly causing skin aging.

Physical aging is mainly reflected by shortening of the cellular telomeres. It has been reported that telomeres shortening is accelerated if the skin is long-term subjected to ultraviolet irradiation by the sunlight, and thus the progress of skin aging is promoted. The telomeres of human being are located at the ends of the chromosomes of the eukaryotic cells, and are constituted by telomere binding protein and tandem repeated DNA sequence 5'-(TTAGGG)-3'. The function of the telomeres is to maintain the stability of the structure and functionality of the chromosomes, prevent the end of the DNA from being degraded or fused, and adjust the normal growth of the cells. The protein encoded by the CTC1 gene is an important constitution element of a telomere binding protein CST composite and is critical to normal duplication of the telomeres and maintenance of the length of the telomeres.

With deep studies on epigenetics in recent years, people have gained new knowledge about how the external factors act on the expression of the human genes. In various epigenetic mechanisms, great importance is placed on microRNA-concerning studies.

The microRNA is a small molecule commonly present in living organisms, with a length of 17 to 27 nucleotides. The microRNA, in combination with a complementary mRNA, may specifically inhibit expression of a target gene. Studies reveal that thousands of human protein-encoding genes is regulated by the microRNA, which indicates that the microRNA is a primary regulator in many important biological progresses. The microRNA is named based on the time sequence when being discovered. Using hsa-miR-1290 as an example, "hsa" indicates that the molecule pertains to human being, "miR" indicates an identifier of the microRNA of a matured body, "1290" indicates a serial number assigned according to the sequence when family members to which the microRNA pertains are discovered or when the microRNA is loaded to the public microRNA database. TargetScan is a public database which studies mutual effects between the microRNAs and the genes, and a target gene may be predicted based on the nucleotide sequence complementation degree of a given microRNA molecule to find a potential target for mechanism study.

### SUMMARY

The present invention is intended to provide a method for screening active substances protecting telomeres in skin cells to develop an external skin preparation for improving skin aging.

The present invention solves the above problem by employing the following technical solution:

According to the present invention, a critical microRNA molecule of skin aging is obtained by comparing skin subjected to ultraviolet irradiation with skin free of ultraviolet irradiation.

The microRNA molecule is hsa-miR-1290, wherein an expression level of the hsa-miR-1290 in the skin subjected to ultraviolet irradiation is substantively upregulated.

Further, the hsa-miR-1290 is an inhibitor of the telomere protective factor CTC1 in skin cells.

The active substances according to the present invention may inhibit expression of the hsa-miR-1290 in the skin cells, and promotes expression of CTC1 gene by gene prediction based on TargetSan and subsequent aging experiment verification, such that the telomeres in skin cells are prevented from being damaged and an effect of improving skin aging is achieved.

According to the present invention, the external skin preparation is a cosmetic product or a skin care product. The cosmetic product or skin care product may be a fundamental face care cosmetic product, a hair care cosmetic product, a body care cosmetic product, a sunscreen cosmetic product or the like, and formulation thereof is not specifically limited. Reasonable choice may be made according to different needs.

According to the present invention, the external skin preparation may further include other active ingredients. The other active ingredients are preferably one or more of a moisturizing active ingredient, an anti-oxidation active ingredient, an oil control active ingredient, a whitening active ingredient and an anti-aging active ingredient. The other active ingredients have a content that is customary in the art.

In one or more specific embodiments of the present invention, the method for screening an active substances protecting telomeres in skin cells according to the present invention is performed by using microRNA in skin cells as a test basis. Preferably, the skin cells are selected from one or more of dermal fibroblast cells and epidermal keratinocytes. Preferably, the microRNA is has-miR-1290. The hsa-miR-1290 has a sequence of SEQ ID NO. 1. The hsa-miR-1290 is an inhibitor a telomere protection factor CTC1 in the skin cells.

In one or more specific embodiments of the present invention, an average expression level of the hsa-miR-1290 in the skin cells in the treatment of the active substances is compared with an average expression level of the hsa-miR-1290 in skin cells in a control group by RT-PCR method. Preferably, in the RT-PCR method, the expression level of the hsa-miR-1290 in the skin cells is measured by 2^{-ΔCt}. More preferably, in the RT-PCR method, the expression level of the hsa-miR-1290 in the skin cells is measured by 2^{-ΔCt}.

In one or more specific embodiments of the present invention, the active substance is configured to develop an external skin preparation for improving skin aging. The external skin preparation is selected from the group consisting of one or more of a face care product, a makeup product, a hair care product and a body care product.

In one or more specific embodiments of the present invention, the active substance is one selected from the group consisting of a pomegranate extract containing composition, a rose oil, a bamboo seed extract, a dihydroflavone derivative, a cudrania tricuspidata extract, a desert plant mixture, a snow chrysanthemum extract, a bamboo leach extract, a peach gum aqueous solution, a peony extract microemulsion, a stachyurus chinensis extract, a fragrant wood extract, a phellinus linteus polysaccharide extract, a composite seed extract, a composite flower extract, a prinsepia utilis Royle extract, a saccharomyces cerevisiae extract, a ginseng seed extract, a leontopodium haplophylloides extract, a peony seed extract, a gentian extract, a limonium sinense Kuntze extract, an Elaeagnus umbellata Thunb. extract, a tender leaf extract of prinsepia utilis, a pomegranate extract containing composition, a snow ginseng extract containing composition, a snow chrysanthemum extract containing composition, a rosa davurica pall extract, a garden balsam extract, a green bonnie flower extract and an acacia extract, or a composition of one or more thereof.

### BRIEF DESCRIPTION OF THE DRAWINGS

FIG. 1 illustrates the difference between expression levels of hsa-miR-1290 in skin subjected to ultraviolet irradiation and in skin free of ultraviolet irradiation ("**" indicates P < 0.01);
FIG. 2 illustrates the difference between expression levels of hsa-miR-1290 in a non-treated reconstructed aging skin model and a reconstructed aging skin model subjected to the treatment by the composition containing pomegranate extract, respectively ("*" indicates P < 0.05);
FIG. 3 illustrates the difference between expression levels of CTClin a non-treated reconstructed aging skin model and a reconstructed aging skin model subjected to the treatment by the composition containing pomegranate extract, respectively ("*" indicates P < 0.05); and
FIG. 4 illustrates the histological staining results of a non-treated reconstructed aging skin model and a reconstructed aging skin model subjected to the treatment by the composition containing pomegranate extract.

### DETAILED DESCRIPTION

For better understanding of the present invention, hereinafter the present invention is further described with reference to some embodiments and accompanying drawings. However, the embodiments of the present invention are not limited hereto. Experimental methods with specific conditions unstated in the following embodiments are routine methods with customary conditions that are readily known by a person skilled in the art, or carried out in accordance with the specifications of relevant products.

In the following examples, an active substance used is a pomegranate extract containing 1.0% of pomegranate extract and 99.0% of glacial water. The pomegranate extract is prepared by the following preparation method: using sour pomegranate from Yuxi, Yunan Province, separating the peel and seeds of the pomegranate, grinding the pomegranate peel into coarse powder with a particle size of 40 to 100 meshes, adding 8 to 12 times of volume of 50 to 90% of ethanol and performing ultrasonic extraction for two times each of which lasts 2 hours, combining filtrates and condensing to 150 mL, enriching via an AB-8 macroporous absorptive resin, and eluting with water, 30% ethanol and 70% ethanol sequentially, collecting a part eluted with the 70% ethanol, and concentrating to dryness to obtain a pomegranate extract. The glacial water is originated from the Dorji Qudengnima Spring in the Himalayas (5128 meters above the sea level); each liter of the glacial water contains the following components: Sr⁺ 0.2-0.5 mg, K⁺ 0.5-10.0 mg, Na⁺ 1.0-5.0 mg, Ca²⁺ 14.0-30.0 mg and Mg²⁺ 2.0-10.0 mg; a total content of a soluble solid is 60 to 150 mg/L; a pH value of the glacial water is 7.4; and a deuterium content in the glacial water is 132 ppm. A composition containing pomegranate extract is prepared by mixing the pomegranate extract with the glacial water.

In a specific example of the present invention, the used active substance is one selected from the group consisting of a rose oil, a bamboo seed extract, a dihydroflavone derivative, a cudrania tricuspidata extract, a desert plant mixture, a snow chrysanthemum extract, a bamboo leach extract, a peach gum aqueous solution, a peony extract microemulsion, a stachyurus chinensis extract, a fragrant wood extract, a phellinus linteus polysaccharide extract, a composite seed extract (extracted via a solvent from peony seeds, lotus seeds, rose seeds, white camellia seeds, jasmine seeds, peach flower seeds, plum seeds, ginseng seeds, harba dendrolii nobilis seends, and fragrans seeds), a composite flower extract (extracted via a solvent from peony flowers, lotus flowers, rose flowers, white camellia flowers, jasmine flowers, peach flowers, ginseng flower, harba dendrolii nobilis flower and fragrans flower), a prinsepia utilis Royle extract, a saccharomyces cerevisiae extract, a ginseng seed extract, a leontopodium haplophylloides extract, a peony seed extract, a gentian extract, a limonium sinense Kuntze extract, an Elaeagnus umbellata Thunb. extract, a tender leaf extract of prinsepia utilis, a pomegranate extract containing composition, a snow ginseng extract containing composition, a snow chrysanthemum extract containing composition, a rosa davurica pall extract, a garden balsam extract, a green bonnie flower extract and an acacia extract, or a composition of one or more thereof, which achieves an protective effect on the telomeres in skin cells to some extent (statistically remarkable or not remarkable).

### Example 1: Testing on natural expression levels of hsa-miR-1290 in aged skin and normal skin

### I. Acquisition of skin samples

Fresh skin samples are purchased from Shanghai Outdo Biotech Co., Ltd., and donors are in the range of between 30 and 40 years old and are health females born and resident in Shanghai. Aging skin samples are originated from face or pouch-part skin that is long-term subjected to ultraviolet irradiation, and totally 8 samples are used. Normal skin samples are originated from belly or chest-part skin that is not subjected to long-term ultraviolet irradiation, and totally 8 samples are used.

Specifically, skin tissues were collected upon the orthopaedic surgery, and the skin tissues were treated as soon as possible. The skin tissues were sheared, with a thickness not exceeding 0.5 cm, into small tissue pieces with a dimension of 0.5 cm × 1 cm × 1 cm (thickness x length x width); the small tissue blocks were placed into sterile Cryo tubes; 2 ml of RNAlater (Sigma) was added to each tube to immerse the tissue for storage; and one skin tissue block was placed into one Cryo tube and the Cryo tube was stored in a low-temperature refrigerator at -80°C.

### II. Total RNA extraction from the skin samples

Ordinary tissues for use in extraction, and a mirVana™ miRNA Isolation Kit without phenol of the cell miRNA were used, and the total RNA extraction was carried out for the samples in accordance with the standard operation procedures provided by the manufacturers. The total RNA obtained through extraction was subjected to quality inspection by Agilent Bioanalyzer 2100 (from Agilent Technologies) and the qualified total RNA was set aside as a reserve.

### III. RT-PCR test

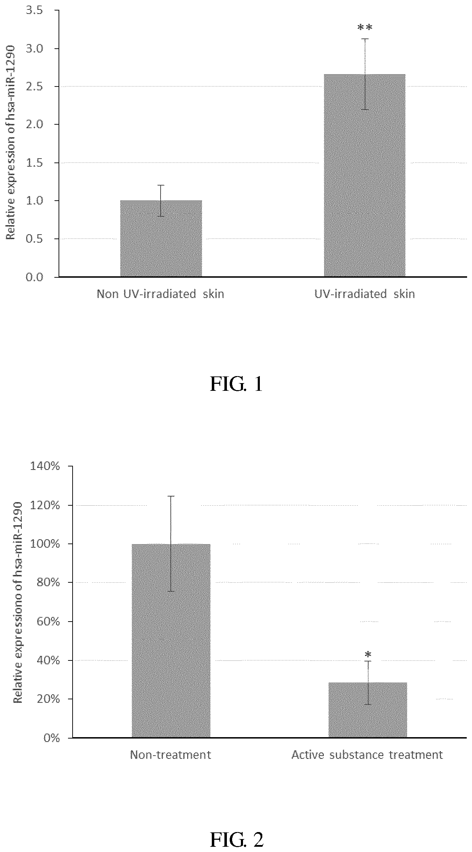

The total RNA of the skin samples was reverse-transcribed into cDNAs by a ReverTra Ace qPCR Kit (from TOYOBO), then an RT-PCR reaction was carried out, and the expression level of hsa-miR-1290 was tested, with U6 as an internal reference. The reaction system is operated in accordance with an instruction manual of a Power SYBR Green PCR Master Mix (from ABI), and each skin sample was loaded in triplicate to a 384 well-plate. FIG. 1 illustrates the result that the expression level of hsa-miR-1290 in the skin subjected to ultraviolet irradiation is substantively higher than that of hsa-miR-1290 in the skin subjected to no ultraviolet irradiation.

### Example 2: Test of impacts caused by an active substance on hsa-miR-1290 in a reconstructed aging skin model

### I. Extraction of aged skin cells

By using purchased fresh face skin sample of a 46-year-old female donor, primary cell isolation was carried out for dermal fibroblast cells and epidermal keratinocytes in a sterile environment. The skin was sterilized with iodide and 75% alcohol, and washed with PBS. Subcutaneous adipose tissues and vessels were trimmed with scissors. The skin was cut into small blocks with a dimension of 0.5 cm × 0.5 cm and digested at 4°C for 15 hours with Dispase II (from Roche). The epidermal layer and the dermal layer were isolated. The dermal layer was digested with 0.05% trypsin(from Invitrogen) for 13 minutes and centrifuged for 10 minutes at a rotation speed of 1000 rpm, the supernatant was discarded, and the cells were resuspended with a K-FSM(from Invitrogen) and then seeded into a 162 cm² flask and placed in a cell incubator at 37°C, 5% CO₂, and saturated humidity for cultivation. When the cell layer grew until 70% to 80% confluent, the cells were collected and frozen to establish a dermal fibroblast cell bank. The epidermal layer was digested with collagenase A (from Roche) for 4 hours and centrifuged for 10 minutes at a rotation speed of 1000 rpm, the supernatant was discarded, the cells were resuspended with DMEM(from Invitrogen) containing 10% FBS, and the primary cultivation was carried out for the cells and the cells were frozen to establish an epidermal keratinocyte bank.

### II. The composition containing pomegranate extract acted on the reconstructed aging skin model.

The dermal fibroblasts and the epidermal keratinocytes were cultivated in vitro and seeded onto a biocompatible scaffold material for 3D cultivation, and 6 photo-aged reconstructed skin models were thus established. The cells were cultivated in groups 3 days after the seeding, 3 skin models in the control group were cultivated by using a common cultivation medium, and the cells in the treatment group were cultivated by using a common cultivation medium containing 2% of the composition containing pomegranate extract. The 3D cultivation was stopped till the 42^{th} day.

### III. Test on an expression of hsa-miR-1290 in a photo-aged reconstructed skin model by RT-PCR

The method is the same as storage of the skin samples, extraction of the total RNA and the RT-PCR process. FIG. 2 illustrates a result, which reveals that the expression level of hsa-miR-1290 in the photo-aged reconstructed skin treated by the active substance is substantively lowered than the group not subjected to the treatment by the active substance. This indicates that the active substance is capable of reversing the hsa-miR-1290 whose expression level is normally increased in the photo-aged skin.

### IV. Verification of CTC1 gene expression in the photo-aged reconstructed skin models by RT-PCR

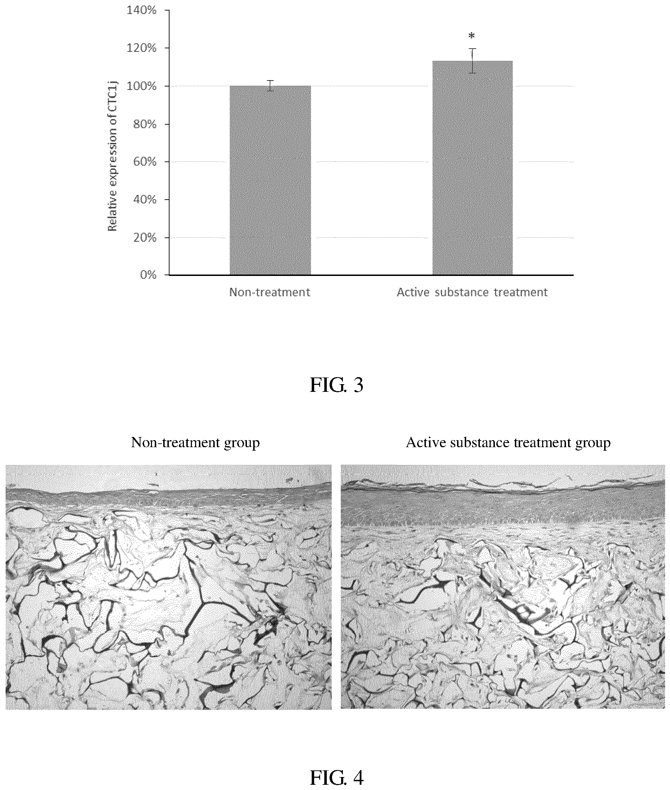

The target gene of hsa-miR-1290 was predicted to be the CTC1 gene by using the TargetScan Release 7.1 online database. A difference of the CTC1 gene expression was compared between the non-treated group and the group treated by the active substance, with 18S as an internal reference. FIG. 3 illustrates a result, which reveals that in the photo-aged reconstructed skin model subjected to treatment by the active substance, the expression level of CTC1 in the skin cells are substantively higher than the non-treated group. This result complies with the prediction.

### Example 3: Effect of the active substance on the structure of the photo-aged reconstructed skin model

In the established photo-aged reconstructed skin model in the above example, on the 42^{th} day when the 3D cultivation was stopped, the skin models in the group subjected to treatment by the active substance and in the group not subjected to treatment by the active substance were collected and placed to a 10% formalin solution for fixation and paraffin embedding, and then cut into thin slices in a thickness of 5 µm. The slices were histologically stained by using Masson's Trichrome Staining method, and microscopic images were taken by using a microscope. FIG. 4 illustrates the result, which reveals that the entire tissue structure of the photo-aged reconstructed skin model not subjected to treatment by the active substance is poor, whereas the photo-aged reconstructed skin model subjected to treatment by the active substance exhibits a complete tissue structure and has a well-organized stratum basale of epidermis, an adhesive layer with improved thickness, and a well-differentiated stratum corneum.

According to the present invention, through the photo-aged reconstructed skin model, it is found that the composition containing pomegranate extract is capable of substantively inhibiting hsa-miR-1290 in the photo-aged skin, such that expression of the CTC1 gene protecting the telomeres in skin cells is promoted. From the histologicalstaining result, it is significant that the compound improves the photo-aged skin.

Described above are merely some exemplary examples for illustrating the present invention, rather than limiting the present invention. A person skilled in the art would derive various equivalent variations or replacements without departing from the inventive spirit of the present invention. These variations or replacements are all within the protection scope defined by the appended claims.

## Claims

1. A method for screening an active substance for protecting telomeres in skin cells, wherein the method is based on detection of microRNA in skin cells.

2. The method according to claim 1, wherein the skin cells are selected from one or more of dermal fibroblasts and epidermal keratinocytes.

3. The method according to claim 1 or 2, wherein the microRNA is hsa-miR-1290.

4. The method according to claim 3, wherein the hsa-miR-1290 has a sequence of SEQ ID NO. 1, and the hsa-miR-1290 is an inhibitor of a telomere protection factor CTC1 in the skin cells.

5. The method according to any one of claims 1 to 4, wherein an average expression level of the hsa-miR-1290 in the skin cells subjected to treatment of the active substance is compared with an average expression level of the hsa-miR-1290 in the skin cells in a control group by using an RT-PCR method.

6. The method according to claim 5, wherein in the RT-PCR method, the expression level of the hsa-miR-1290 in the skin cells is measured by 2^{-ΔCt}.

7. The method according to claim 6, wherein a measurement standard of a test result by the RT-PCR method is: whether the expression level of the hsa-miR-1290 in the skin cells subjected to treatment of the active substance is substantively reduced relative to the control group (P < 0.05).

8. The method according to claim 7, wherein the active substance is used to develop an external skin preparation for improving skin aging.

9. The method according to claim 8, wherein the external skin preparation is selected from the group consisting of one or more of a face care product, a makeup product, a hair care product and a body care product.

10. The method according to claim 7, wherein the active substance is one selected from the group consisting of a pomegranate extract containing composition, a rose oil, a bamboo seed extract, a dihydroflavone derivative, a cudrania tricuspidata extract, a desert plant mixture, a snow chrysanthemum extract, a bamboo leach extract, a peach gum aqueous solution, a peony extract microemulsion, a stachyurus chinensis extract, a fragrant wood extract, a phellinus linteus polysaccharide extract, a composite seed extract, a composite flower extract, a prinsepia utilis Royle extract, a saccharomyces cerevisiae extract, a ginseng seed extract, a leontopodium haplophylloides extract, a peony seed extract, a gentian extract, a limonium sinense Kuntze extract, an Elaeagnus umbellata Thunb. extract, a tender leaf extract of prinsepia utilis, a pomegranate extract containing composition, a snow ginseng extract containing composition, a snow chrysanthemum extract containing composition, a rosa davurica pall extract, a garden balsam extract, a green bonnie flower extract and an acacia extract, or a composition of one or more thereof.
